# EUROPEAN PATENT APPLICATION

(11) **EP 2 366 776 A1**
(43) Date of publication of application: **21.09.2011**
(21) Application number: 10305206.4
(22) Date of filing: 01.03.2010
(51) Int. Cl.: C12N 7/04, C12N 15/867, C12Q 1/18, C12Q 1/70

(54) **A method for measuring viral infectivity**

(71) Applicant: Epixis, 75013 Paris (FR)
(72) Inventor: Dalba, Charlotte, 75013, Paris (FR); Garrone, Pierre, 69008, Lyon (FR); Fluckiger, Anne-Catherine, 69130, Ecully (FR); Du Chene, Isaure, 69007, Lyon (FR); Mancip, Jimmy, 69009, Lyon (FR)
(74) Representative: Chajmowicz, Marion

(57) **Abstract**

The invention relates to an *in vitro* method for evaluating a test substance for its ability to modulate, preferably to inhibit, infectivity of an enveloped virus, by using preparations containing retroviral particles coding at least two different reporter genes, one suitable to measure the infectious titer of the particle preparations and the other suitable to measure the global level of infection.

## Description

The present invention relates to *an in vitro* method for evaluating a test substance for its ability to modulate infectivity of an enveloped virus, using a retrovirus-like particle carrying a multiple reporter gene vector.

### Technical Background:

*In vitro* assays are crucial tools to study the infectivity of a virus as well as to evaluate test substances capable of interfering with this infection. For instance neutralization assays have been developed to study the immune response in infected individuals, monitor viral diversity and judge vaccine immunogen candidates in both clinical and preclinical trials. Furthermore such neutralization assays, and more generally infection assays with viral particles, require optimization and standardization. This is especially important when neutralization activity of a given sample is to be compared against different virus strains or in assays performed at different times across different studies, for instance.

One of the most important parameters for development and standardization of such assays is the amount of virus or virus pseudoparticles (pp) used (see Sirskyj et al, J Virol Methods. 2009 Nov 11). This requires an assay with a read-out system suitable to precisely quantify the infectious titer of each virus or virus pseudoparticle preparation and to define which titer (or dilution of virus preparations) will be used subsequently in the infection or neutralization assays.

Infection and neutralization assays with retroviral particles utilizing read-out systems measuring the precise number of infected cells are suitable to determine the infectious titer of the retroviral pseudoparticle preparations. As example, this can be obtained by counting the number of fluorescent cells by flow cytometry or the number of coloured cells by microscopy after infection of target cells with retroviral particles carrying a retroviral vector coding, for instance, either a green fluorescent protein (GFP) or the β-lactamase/LacZ enzyme (e.g. Negre et al., Gene Therapy, 2000, 7(19):1613-23). However, assays using such read-out systems cannot be easily scaled up for development of rapid, sensitive and quantitative assays measuring activity of a large number of samples or sample dilutions. On the other hand, infection and neutralization assays utilizing read-out systems measuring the global signal obtained from culture of target cells are more suitable to perform rapid testing of large numbers of samples, but are not always suitable to measure the precise infectious titer of the retroviral pseudoparticle preparations. An example of such read-out systems includes the measurement of luminescence activity on a luminometer in lysates of target cells infected with retroviral particles carrying a retroviral vector coding a luciferase reporter gene. See e.g. David C. Montefiori (2009) Measuring HIV Neutralization in a Luciferase Reporter Gene Assay. HIV Protocols, Second Edition, Vol. 485, V.R. Prasad and G.V. Kalpana, eds.

### Summary of the invention:

The invention relates to *an in vitro* method for evaluating a test substance for its ability to modulate, preferably to inhibit, infectivity of an enveloped virus, by using preparations containing retroviral particles coding at least two different reporter genes, one suitable to measure the infectious titer of the particle preparations and the other suitable to measure the global level of infection.

More particularly, the invention provides an *in vitro* method for evaluating a test substance for its ability to modulate, preferably to inhibit, infectivity of an enveloped virus, which method comprises
a) contacting a host cell with a population of recombinant pseudotyped retrovirus particles comprising (i) a replication-defective genome of a retrovirus, (ii) core protein(s) of said retrovirus, and (iii) an envelope that comprises at least one protein from an enveloped virus, wherein said population altogether carries at least two R1 and R2 reporter genes, wherein R1 and R2 are distinct, and measuring infectious titer of said particles by means of the R1 reporter gene;
b) adjusting the dilution of the population of retrovirus particles showing the infectious titer measured in step (a) to reach a defined infectious titer suitable for measuring efficacy of infection, and, in the presence or the absence of the test substance, contacting a host cell with said population of retrovirus particles at said defined infectious titer and measuring efficacy of infection of said cell by said retrovirus particles by means of the R2 reporter gene; and
c) comparing the efficacy of infection in the presence of the test substance with efficacy of infection in the absence of the test substance, whereby the ability of the test substance to modulate infectivity of the retrovirus particle is determined and reflects the ability of the test substance to modulate infectivity of the enveloped virus.

In a preferred embodiment, the reporter genes R1 and R2 are carried by a sole retrovirus genome and preferably are operably linked.

Alternatively the reporter genes R1 and R2 may be carried by two distinct retrovirus genomes.

Advantageously, said reporter gene R1 encodes a gene product suitable for measuring infectious titer of said retrovirus particles, by counting the number of cells transduced by the particles, such as an enzyme, a chromogenic agent, or a fluorescent agent.

Said reporter gene R2 advantageously encodes a gene product suitable for measuring the efficacy of infection of said cell by said retrovirus vector, such as an enzyme, a chromogenic agent, a luminescent agent, or a fluorescent agent.

In a preferred embodiment, step (b) comprises screening one or a plurality of test substances, in parallel or subsequently, with the same population of particles or with different populations of particles.

The invention further provides a recombinant pseudotyped retrovirus particle comprising (i) a replication-defective genome of a retrovirus, (ii) core protein(s) of said retrovirus, and (iii) an envelope that comprises at least one protein from an enveloped virus wherein said genome includes at least one reporter gene R1 and at least one reporter gene R2, wherein R1 and R2 are distinct and preferably are operably linked.

Another subject of the invention is a composition comprising a population of recombinant pseudotyped retrovirus particles comprising
- particles comprising (i) a replication-defective genome of a retrovirus, (ii) core protein(s) of said retrovirus, and (iii) an envelope that comprises at least one protein from an enveloped virus, wherein said genome includes at least one reporter gene R1,
- and particles comprising (i) a replication-defective genome of said retrovirus, (ii) core protein(s) of said retrovirus, and (iii) an envelope that comprises at least said protein from said enveloped virus, wherein said genome includes at least one reporter gene R2, distinct from R1,
in association with a vehicle.

The population may further comprises such recombinant pseudotyped retrovirus particles comprising a genome that includes at least one reporter gene R1 and at least one reporter gene R2, distinct from R1, where R1 and R2 are operably linked.

The invention is directed to the use of such particles or such composition for evaluating a test substance for its ability to modulate, preferably to inhibit, infectivity of an enveloped virus.

### Legends to the Figures:

**Figure 1A** is a schematic representation of double-reporter MLV-based retroviral vectors.
**Figure 1B** is a schematic representation of an example of double-reporter MLV-based retroviral vector encoding a polyprotein GFP-2A-Luciferase. LTR: Long Terminal Repeat. CMV: CytoMegaloVirus. PBS: Primer Binding Site. PPT: PolyPurine Tract. Ψ: Packaging sequence.

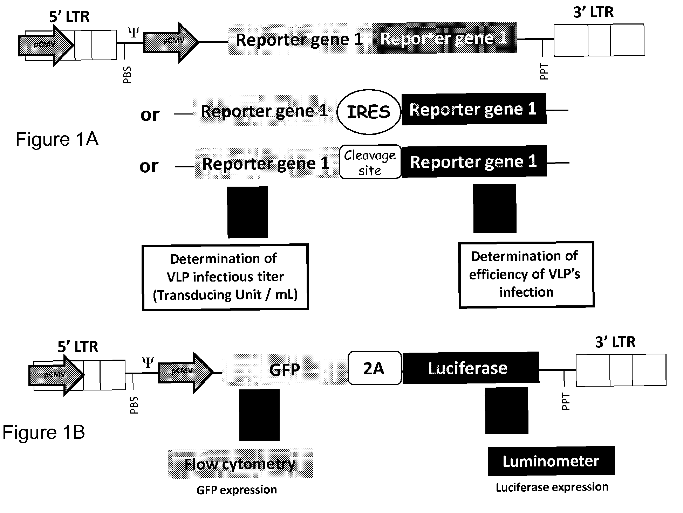

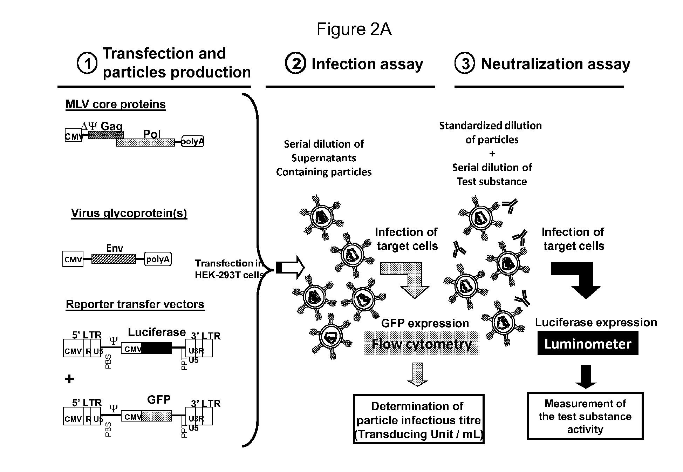

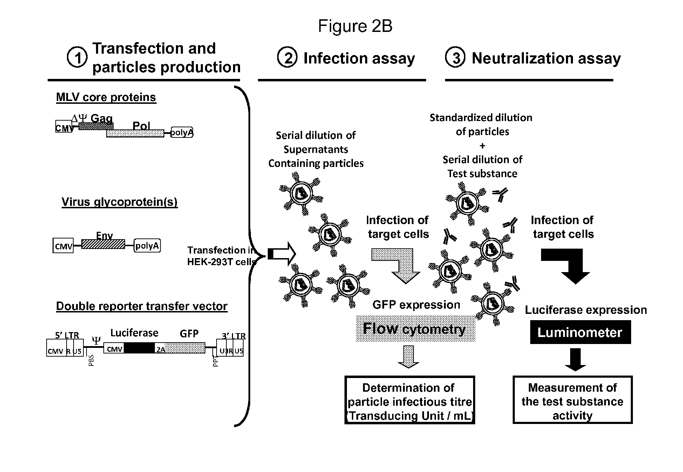

**Figures 2A** and **2B** show a schematic representation of a particle production, an infection assay and a neutralization assay using retroviral pseudoparticles that contain either two single-reporter MLV-based retroviral vectors encoding GFP or Luciferase, respectively (**Figure 2A**), or one double-reporter MLV-based retroviral vector encoding a polyprotein GFP-2A-Luciferase (**Figure 2B**).

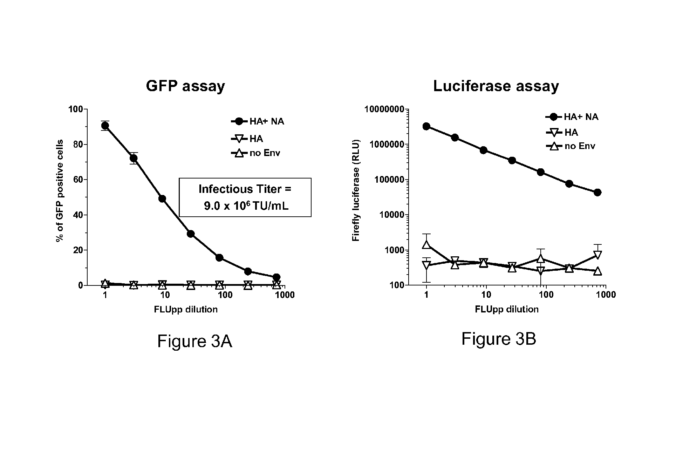

**Figure 3A and 3B** are graphs that show infectivity of retroviral pseudoparticles H5N1-FLUpp (FLUpp = retroviral pseudoparticles pesudotyped with enveloped proteins from an influenza (Flu) virus) expressing hemagglutinin (HA) and neuraminidase (NA) from avian Influenza A/Vietnam/1194/2004 HSN1 virus by measuring percentage of GFP-positive target cells (**Figure 3A**) and luciferase activity (**Figure 3B**).

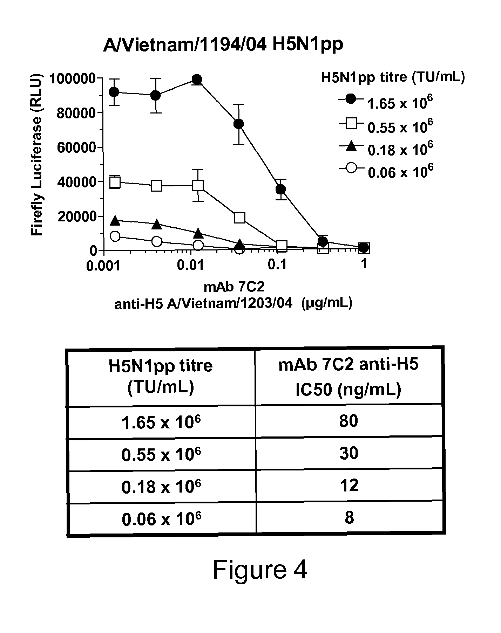

**Figure 4** is a graph that shows neutralization of H5N1-FLUpp derived from A/Vietnam/1194/2004 virus and used at different titers with monoclonal antibody (mAb) against HA (mAb 7C2) by measuring luciferase activity in target cells.

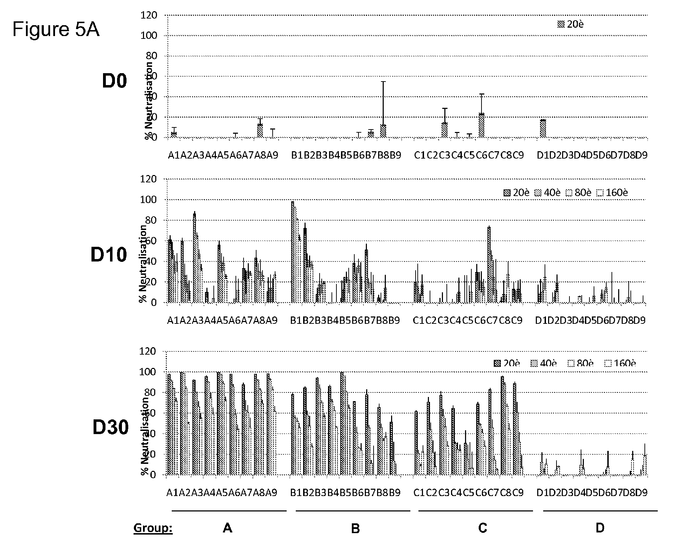

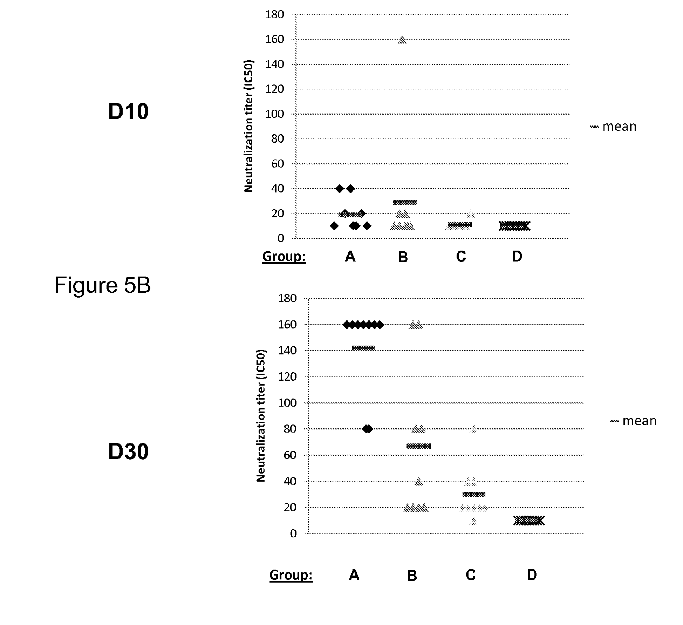

**Figure 5A** shows neutralization of H5N1-FLUpp from A/Vietnam/1194/2004 virus by dilutions of sera from mice immunized with DNA candidate vaccines against avian Influenza H5N1 A/Vietnam/1194/2004 virus at Day 0 (D0), Day 10 (D10) and Day 30 (D30). Luciferase activity was measured in target cells and results are expressed as mean +/- SD of triplicates of the percentage of neutralization relative to incubation of H5N1-FLUpp in the absence of sera. **Figure 5B** shows neutralization titers (IC50) of mouse sera at D10 and D30. Results are expressed as neutralization titer defined as the highest dilution that reaches 50% of neutralization relative to incubation of H5N I -FLUpp without serum.

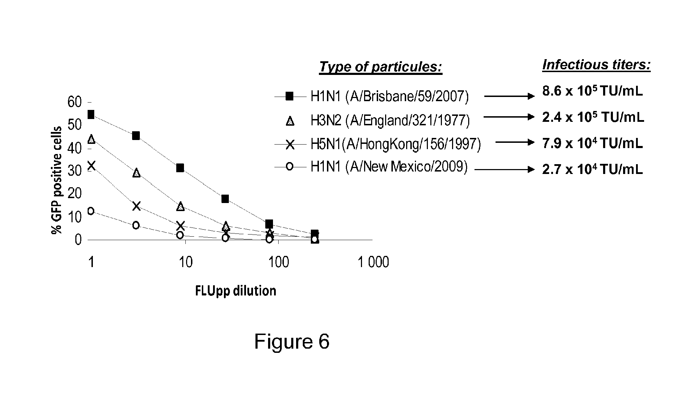

**Figure 6** is a graph that shows the measurement of the infectious titer of various FLUpp carrying a double-reporter vector on MDCK target cells by flow cytometry. FLUpp were pseudotyped with HA and NA from either H1N1 pandemic A/New Mexico/2009, H1N1 seasonal A/Brisbane/59/2007, H3N2 A/England/321/1977 and HSN1 A/HongKong/156/1997.

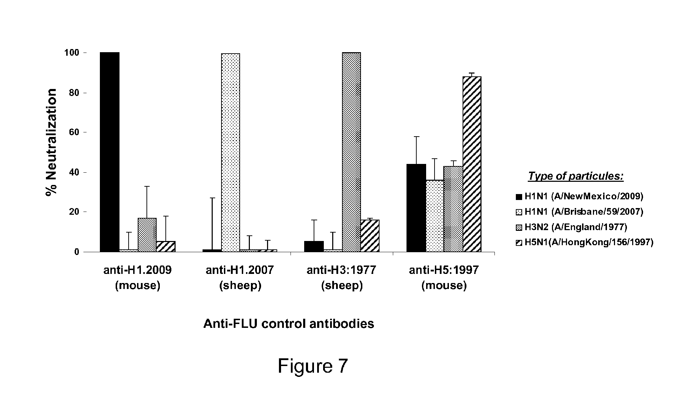

**Figure 7** shows the neutralization activity of optimal dilutions of various antisera when tested against the indicated preparations of particles carrying a double-reporter gene and used at the same infectious titer of 1 x 10⁴ TU/mL. The following antiserum were used: antiserum against HA from H1N1 A/NewMexico/2009 (anti-H1:2009, mouse serum produced in the laboratory), from IVR148 (sheep anti-H1:2007, NIBSC 08/112), from H3N2 A/England/321/77 (sheep anti-H3:1977, NIBSC 78/569), from H5N1 A/HongKong/156/1997 (mouse anti-H5:1997 serum produced in the laboratory).

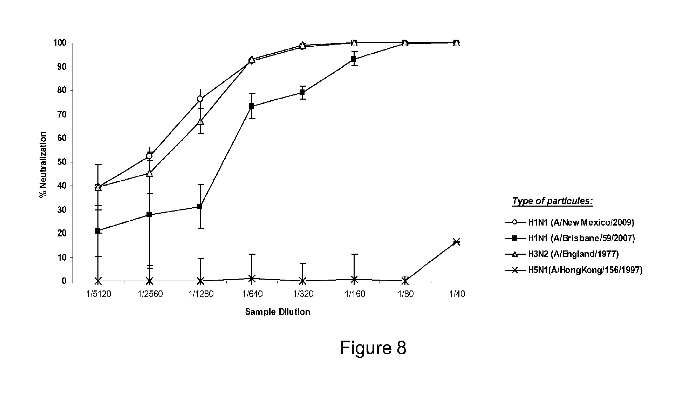

**Figure 8** shows the neutralization activity of a pool of human sera recommended as standard for antibody to influenza HINlv (pandemic variant 2009) virus (NIBSC 09/194) against the indicated FLUpp carrying a double-reporter gene and used at a same infectious titer of 1 x 10⁴ TU/mL.

### Detailed description of the invention:

In order to standardize infection or neutralization assays with pseudotyped retroviral particles, the inventors have optimized the production of infectious viral particles to allow both the determination of the infectious titer and the running of rapid and large numbers of quantitative neutralization assays with the same pseudoparticle preparations. This is achieved by using retroviral particle preparations containing retroviral vectors coding at least two different reporter genes, one suitable to determine the infectious titer of the particle preparations by measuring the number of infected cells (a gene coding a GFP, for example) and the other suitable to measure the overall levels of infection in the whole target cell population (a gene coding a luciferase enzyme allowing measurement of luciferase activity in lysates of target cells, for example). The two reporter genes can be introduced *in trans* by co-transfection of the retroviral particle producer cells with two different packaging-competent retroviral vectors encoding each one of the reporter genes. Or, preferably the two reporter genes will be introduced *in cis* by transfection of the producer cells with a unique packaging-competent retroviral vector containing a unique cassette encoding the two reporters, so that the nucleotide sequences encoding the two reporters will be packaged equally within infectious retroviral particles. This can be achieved, for example, by using bicistronic cassettes or cassettes coding for the two reporters expressed as polyproteins further cleaved by protease activity in infected cells or expressed as fusion proteins in infected cells.

This invention is described in greater details below.

### The retrovirus particles

The expression *"recombinant pseudotyped retrovirus particle* "designates synthetic non-naturally occurring viral particles typically produced by genetic engineering or synthesis, *in vitro, ex vivo* or *in vivo.* The terms "virus-like particles" (VLPs), "pseudo-particles" (pp) or "pseudotyped particles" (pp) may be used as well, and are synonymous in the context of the invention. Synthetic pseudo-particles have been described in WO 02/34893, or EP 1 398 371.

The VLP comprises a genome of a retrovirus, also named "retroviral vector". The expression "retroviral vector" designates synthetic modified genome of a retrovirus.

Retroviruses are enveloped RNA viruses (see e.g. Coffin JM (1992) Structure and classification of retroviruses. In The Retroviridae, pp.19-49. ed. JA Levy. New York: Plenum; Goff SP (2001) Retroviridae: the retroviruses and their replication. In Fields' Virology,Vol. 2. pp. 1871-940. Knipe DM, Howley PM, eds. Philadelphia: Lippincott, Williams & Wilkins). It is known that the genomic organization of retroviruses comprises essentially the following elements:
- an LTR ("Long Terminal Repeat") region, located at each end of the genome. Each LTR region is composed essentially of three functional regions termed U3, R and U5,
- a packaging sequence ("Psi"), involved in the packaging of the proviral genome in the viral particle,
- three coding regions, designated gag, pol and env, respectively coding the core proteins (capsid, matrix and nucleocapsid), the enzymes (reverse transcriptase, protease, integrase) and the envelope glycoproteins.

In the case of lentiviruses, their genome further comprises additional coding or regulatory sequences, such as vif, vpr, vpu, vpx, rev, tat, and nef.

Generally, the recombinant vectors used in the present invention comprise at least a packaging sequence ("Psi"), as well as 5'-LTR and 3'-LTR, used as wild-type or modified sequences. Preferably, the particles are produced by replacing viral proteins encoding sequences (gag/pol/env) with the reporter genes.

In the context of the invention, the recombinant pseudo-particles are "infectious" in that they can complete the initial steps of viral cycle that lead to cell entry, followed by retrotranscription of viral RNA into dsDNA by viral enzymes and integration into the host cell genome. However, upon interaction with the host cell, the particles may or may not produce progeny viruses.

In the context of the invention, the term "retroviral genome" means that the particle comprises a nucleic sequence comprising a packaging competent retrovirus-derived genome, i.e. a sequence that comprises the retroviral nucleic acid sequences known as "cis-acting" sequences. These include the LTRs for the control of transcription and integration, the psi sequence necessary for encapsidation, and the Primer Binding site (PBS) and polypurine track (PPT) sequences necessary for reverse transcription of the retroviral genome.

In a preferred embodiment, the genome is a "replication-defective genome of a retrovirus", which is a genome that is not capable of replication in the absence of trans-complementing functions, and thus lacks at least one functional retroviral gene necessary for replication. Preferably, the gag, pol and env genes of the retroviral genome have been deleted so that it cannot replicate.

The VLP further comprises core protein(s) of said retrovirus. As used herein, the term "core protein(s)" denotes a native core protein of a retrovirus, a fragment thereof, or a variant thereof. Generally the core proteins are gag and pol proteins.

The retrovirus particles may be produced, at least partially, from a large variety of retrovirus types and serotypes. The retrovirus may be selected from the group of onco-retroviruses, lentiviruses or spumaviruses. For instance onco-retroviruses can be manipulated easily. Specific examples of onco-retroviruses include Murine Leukemia Virus (MLV), avian leukosis virus (ALV), bovine leukemia virus (BLV), mouse mammary tumour virus (MMTV) or Rous sarcoma virus (RSV) for instance. Lentiviruses represent another class of retroviruses. Their genomic organization has been characterized and can be manipulated to target particular cell populations, notably quiescent cells. Specific examples of lentiviruses include human immunodeficiency virus (HIV), simian immunodeficiency virus (SIV) or equine infectious anemia virus (EIAV), caprine arthritis encephalitis virus (CAEV), for instance. Also spumaviruses including human foamy virus (HFV) can be manipulated to generate retroviral particles.

Preferably the retroviral genome is derived from MLV, ALV, RSV, HIV-1, HIV-2, SIV, EIAV, CAEV, or HFV.

### The pseudotyped envelope of the VLPs:

The VLPs used in the invention comprise an envelope that comprises at least one protein from an enveloped virus, heterologous or not to said retrovirus.

Said enveloped virus is the virus against which infection or neutralization assay is to be performed. As used herein, the term "heterologous virus" refers to a virus that is not a retrovirus, or is a different retrovirus (e.g. a different species or a different strain) than the retroviral genome. Preferably the protein from the enveloped virus derives from the envelope of said virus. Preferably the protein is from a heterologous enveloped virus and derives from the envelope of said heterologous virus. An engineered variant of an endogenous envelop protein is not considered as a protein from a heterologous enveloped virus. The term "heterologous envelope protein" may be used alternatively, referring to a protein or polypeptide that is not native or is not naturally occurring in the retrovirus from which the retroviral genome is derived.

Said envelope protein can comprise a modified (e.g. mutated) form of the natural protein.

These viruses can be either DNA or RNA viruses. Preferably, these viruses are pathogens for humans or animals. Exemplary enveloped viruses amenable to the present invention include, but are not limited to, those selected from *Arenaviridae* (e.g., arenavirus such as lymphocytic choriomeningitis virus), *Bunyaviridae* (e.g., phlebovirus or hantavirus), *Coronaviridae* (e.g., coronavirus or torovirus), *Filoviridae* (e.g., Ebola-like viruses), *Flaviviridae* (e.g., hepacivirus or flavivirus), *Herpesviridae* (e.g., simplexvirus, varicellovirus, cytomegalovirus, roseolovirus, or lymphocryptovirus), *Orthomyxoviridae* (e.g., influenza A virus, influenza B virus, influenza C virus, or thogotovirus), *Paramyxoviridae* (e.g., pneumovirus, morbillivirus, or rubulavirus), *Poxviridae* (e.g., orthopoxvirus, avipoxvirus, or leporipoxvirus), Retroviridae (e.g., lentivirus or spumavirus), *Rhabdoviridae* (e.g., lyssavirus, novirhabdovirus, or vesiculovirus), and *Togaviridae* (e.g., alphavirus or rubivirus).

Preferably, the enveloped virus is a virus of the family of *Arenaviridae* (e.g. Lassa virus), *Coronaviridae* (e.g. Sever Acute Respiratory Syndrome virus), *Flaviviridae* (e.g. Hepatitis C Virus, Dengue virus, West Nil Virus, Yellow Fever Virus, Tick-Borne Encephalitis virus), *Filoviridae* (e.g. Ebola, Marburg), *Herpesviridae* (e.g. Herpes Simplex Virus, Cytomegalovirus, Epstein-Barr Virus, Varicella Zoster Virus), *Orthomyxoviridae* (e.g. Influenza A and B), *Paramyxoviridae* (e.g. Respiratory Syncytial Virus, ParaInfluenza Virus, PMV, Measles), *Poxviridae* (e.g. Vaccinia, Variola), *Rhabdoviridae* (e.g.Vesicular Sstomatitis Virus, Viral Hemorrhagic Septicemia Virus, Rabies), *Retroviridae* (e.g. HIV and other retroviruses), *Togaviridae* (e.g. Chikungunya, Sindbis, Semliki Forest Virus, Ross River Virus, Eastern Equine Encephalitis Virus) .

In a preferred embodiment, the pseudotyped retroviral VLP comprises an envelope that comprises a heterologous glycoprotein, e.g. at least a hemagglutinin (HA) of influenza virus. HA may be of any type, e.g. it may be selected from H1, H3, H5 and H7. The envelope may further comprise neuraminidase (NA) of influenza virus, e.g. selected from N1 or N2. For instance the particle VLP may comprise HSN1, H1N1, H7N1, H3N2, etc.

In another embodiment, said VLP is pseudotyped with proteins of hepatitis C virus (HCV), such as envelope glycoproteins E1 and/or E2. The proteins may be from any HCV genotype, such as e.g. 1a (e.g. H77), 1b (e.g. Strain A, CGlb, Conl), 2a (e.g. JFH-1), 2b (e.g. UKN2B-1.1), 4c (e.g. UKN4-11.1).

In still another embodiment, said VLP is pseudotyped with proteins of HIV, such as glycoproteins gp120 or gp4l. The proteins may be from any HIV genotype (e.g. HIV-1, HIV-2).

### The reporter genes:

The invention makes use of reporter genes that are inserted into the retroviral genome of the recombinant VLPs.

More specifically, said reporter gene R1 encodes a gene product suitable for measuring infectious titer of said retrovirus VLPs, such as an enzyme, a chromogenic agent, a luminescent agent, or a fluorescent agent. Preferably R1 encodes a fluorescent agent. In a preferred embodiment, the reporter gene is a fluorescent agent, such as GFP, and the titer of infection is quantified by means of fluorescent cell counting, for instance by flow cytometry analysis of cells contacted with said pseudotyped VLPs.

Said reporter gene R2 encodes a gene product suitable for measuring the efficacy of infection of said cell by said VLPs, such as an enzyme, a chromogenic agent, a luminescent agent, or a fluorescent agent. Preferably R2 encodes a luminescent agent.

R1 and R2 are distinct genes.

Examples of useful enzymes include beta-lactamase, beta-galactosidase, alkaline phosphatase, SEAP (secreted alkaline phosphatase). Examples of fluorescent agents include green, red or yellow fluorescent proteins. Examples of luminescent agents include luciferase proteins (such as firefly, renilla, gaussia luciferase). When appropriate, antibiotic resistance gene product may be used as a reporter gene, such as those conferring blasticidine, genticine, hygromycine, or neomycin resistance. A cell surface marker that can be detected by an antibody may also prove useful as a reporter gene.

In a preferred embodiment, said reporter gene R1 encodes Green Fluorescent Protein (GFP) and said reporter gene R2 encodes luciferase, especially firefly luciferase..

### The construction of the retrovirus particles:

In a preferred embodiment, the particles as defined in step (a) are a sole particle that includes both reporter genes R1 and R2, which preferably are operably linked.

Alternatively, the particle including reporter gene R1 is distinct from the particle including reporter gene R2.

When the same particle comprises R1 and R2 genes, various constructions are encompassed.

Preferably R1 and R2 are operably linked, which generally means that their expression is controlled by the same promoter (**Figure 1A**).

In the host cell, the 5'-LTR of the integrated retroviral genome may drive the expression of the transgene encoding R1 and R2 reporters. Preferably, transgene expression is driven by an internal, heterologous promoter that allows constitutive expression of R1 and R2 in a cell-type restricted manner.

The promoter may be a constitutive promoter. Examples of suitable promoters include eukaryotic promoters such as human cytomegalovirus (CMV) promoter and its early promoter, simian virus SV40 promoter (EF1α), and the chicken cytoplasmic β-action promoter.

In one embodiment, said reporter genes R1 and R2 are in the form of a monocistronic RNA or of a bicistronic RNA, preferably comprising an internal ribosome entry site (IRES) between the two reporter genes.

In another embodiment, said reporter genes R1 and R2 encode a single fusion protein.

In another embodiment, said reporter genes R1 and R2 encode a single polyprotein that comprises a site of enzymatic cleavage, or an auto-cleavage site from a known viral sequence. Examples of sequences of said sites can derived from positive strand RNA viruses encoding polyproteins that are cleaved by viral or host proteinases to produce mature proteins (see Ryan M.D. and Flint M., 1997, J.. Gen. Virol., 78:699-723; Szymczak A.L. and Vignali D.A., 2005, Expert Opin. Biol. Ther., 5:627-638).

For instance one can insert a 2A polypeptide cleavage site of the foot-and-mouth disease virus (FMDV), located between the translation product of R1 and the translation product of R2. Such FMDV 2A cleavage sequence is well known to any one skilled in the art of constructing retroviral vectors. The FMDV 2A peptide is a 18 amino acid peptide that is used to avoid the need of proteinases to process a polyprotein (see e.g. Pablo de Felipe, et al 1999, Gene therapy, 6:198-208; Pablo de Felipe, 2004, Genetic Vaccines and Therapy, 2:13; Dhanalakshmi Chinnasamy et al, 2006, Virology Jounal, 3:14; Abdelilah Ibrahimi et al, 2009, Human Gene Therapy, 20:845-860).

In a preferred embodiment, the particle comprises a genome carrying a sequence that encodes a GFP-(FMDV2A cleavage site)-Luciferase polyprotein **(Figure 1B)**.

### The production of the pseudotyped retroviral particles:

The VLPs may be prepared according to techniques known in the art. In particular, they may be produced using conventional packaging cells or through transient or stable transfection. Typical methods of producing retroviruses *in vitro* are known, and disclosed for instance in WO89/07150, WO90/02806, US 4,861,719, WO98/02529. Typically, the production comprises culturing a packaging cell and collecting the produced particles. More particularly one may follow the protocols described in Bartosch, et al 2003, J. Exp. Med. 197:633-642; or Hsu, et al, 2003, Proc. Natl. Acad.Sci.USA 100:7271―7276.

The packaging competent cells are co-transfected preferably with a double-reporter retroviral vector for expression of both R1 and R2, a core protein vector, and an envelop protein vector; Alternatively, the packaging competent cells are co-transfected with two single-reporter retroviral vectors for expression of R1 and R2 respectively, a core protein vector, and an envelope protein vector (see **Figure 2A** **and** **2B**).

In a preferred embodiment, the pseudotyped recombinant retrovirus vector is a packaging competent (Psi+) MLV-based vector that encodes CMV-driven expression of a single polyprotein GFP-2A-Luciferase further cleaved at the 2A polypeptide cleavage site introduced between the two proteins, **(see Figures 1A and 1B).**

### Standardized infection and neutralization assays:

The VLPs described herein are useful in *an in vitro* method for evaluating a test substance for its ability to modulate infectivity of an enveloped virus.

More particularly, the invention makes use of the VLPs pseudotyped with proteins from an enveloped virus as defined herein, for the identification of susbtances capable of interfering with the entry of said enveloped virus into cells.

The test substance is any natural, synthetic, or biological compound. Said substance may be any molecule, including small chemical molecules, predetermined or not defined, optionally in a mixture, or an antibody, or any sample, including any biological sample, such as serum or blood.

In particular, herein is provided a method of screening or identification of molecules or substances capable of interfering with entry of an enveloped virus into a cell comprising comparing the level of cell infection by the particles of the invention in the presence or the absence of a test molecule or substance.

The method of the invention comprises taking into account the infectious titer determined in step (a) to perform the tests of step (b), it being understood that step (a) does not need performing more than once for the same retrovirus particle preparation, while step (b) may be run as many times as desired, in the presence of one or of a plurality of test substances, with one or a plurality of different VLPs.

According to said method, the concentration of retrovirus particles showing the infectious titer measured in step (a) is adjusted by dilution, so that it reaches a defined infectious titer."Adjusting the dilution" means concentrating or diluting the particles (i.e. increasing or reducing the infectious titer). The term "defined infectious titer" means an infectious titer of reference. It is predetermined, generally empirically, to allow sufficient sensitivity for the test of step (b), that is testing the efficacy of infection, to be readable. Accordingly one can observe a difference in the efficacy of infection in the presence or absence of a substance that is able to modulate said efficacy of infection.

The method of the invention (as shown e.g. on **Figures 2A** **and** **2B**) preferably comprises the steps consisting of:
- Determining the infectious titer of VLPs pseudotyped with protein(s) from said enveloped virus by contacting a cell susceptible to infection by said enveloped virus with serial dilutions of said VLPs, under conditions that allow cell infection in the absence of any test substance; counting the number of target cells expressing R1 reporter gene product, e.g. GFP by flow cytometry, and calculating the infectious titer of said VLPs preparation;
- Choosing a reference infectious titer for the use of the VLPs, that will be identical for all VLPs to be compared in infection and neutralization assays, e.g, different batches of VLPs pseudotyped with a same virus envelope, or with envelopes from different strains of virus;
- Contacting said cell susceptible to infection by the dilution of said VLPs, that gives said reference titer, in the absence and in the presence of a test substance; assessing cell infectivity in the absence and in the presence of said test substance by measuring the level of expression of R2 reporter gene product, e.g. luciferase activity by luminescence; comparing cell infectivity measured in presence of said test substance with cell infectivity measured in absence of any test substance;
- Identifying as a substance capable of interfering with the entry of said enveloped virus the test substance for which cell infectivity, as measured in the presence of said substance, is altered as compared to cell infectivity measured in the absence of any test substance.

Contacting a host cell with pseudotyped VLPs, and a test substance can be carried out by contacting simultaneously said cell, the VLPs and the test substance. Otherwise, two of these three elements can be contacted under conditions sufficient to allow their interaction before addition of the third missing element.

Cell infectivity or efficacy of infection can be readily assessed by one skilled in the art monitoring the expression of the reporter gene.

The host cell used in the method of the invention generally is a permissive cell, i.e a cell that is susceptible, either naturally or after modification, to infection by an enveloped virus and corresponding synthetic VLPs.

Suitable mammalian host cells include Huh-7 human hepatocellular carcinoma, Hep3B human hepatocellular carcinoma (e.g. ATCC HB-8064), HepG2 human hepatocellular carcinoma (e.g. HB-8065), HT-1080 human fibrosarcoma (e.g. CCL-121), 293T human embryo kidney cells (e.g. ATCC CRL-1573), TE671 human rhabdomyosarcoma (e.g. ATCC CRL-8805), Jurkat human T cell leukemia (e.g. TIB-152), CEM human lymphoblastic leukemia (e.g. CCL-119), COS-7 African green monkey fibroblasts kidney (e.g. CRL-1651), VERO African green monkey kidney (e.g. CCL-81), PG-4 feline astrocyte (e.g. CRL-2032), BHK-21 golden hamster kidney (e.g. CCL-10), CHO Chinese hamster ovary (e.g. ATCC CCL-61), NIH3T3 mouse fibroblasts, and Madin Darby Canine Kidney (MDCK, e.g. ATCC CCL-34).

In a particular embodiment, a suitable host cell for evaluating or screening substances for their ability to modulate infectivity of HCV may be a hepatocyte cell line or a primary human hepatocyte.

In a preferred embodiment, target cells may be Huh-7 human hepatocellular carcinoma cells (Nakabayashi, H., et al, 1982, Cancer, 42:3858) to monitor infection by HCV pseudotyped VLPs.

In another particular embodiment, MDCK (e.g. ATCC-CCL-34) or TE-671 (e.g. ATCC-CRL-8805) cells may be used to monitor infection by Influenza virus pseudotyped VLPs.

In a preferred embodiment, the method of evaluation is a high-throughput screening method.

In particular step of contacting a host cell with a pseudotyped VLP and measuring efficacy of infection of said cell by said VLP, may comprise screening a plurality of test substances, in parallel or subsequently, with the same VLP.

The method may also be industrialised to allow testing crossreactivity of one or multiple test substances by evaluating the effect of the said test substances on a variety of VLPs pseudotyped with a variety of virus envelopes.

The methods described herein are further useful for diagnosis and follow-up of infection by enveloped virus, for instance to assess efficacy of a therapy in a patient, and for prediction of susceptibility to infection by enveloped virus.

The methods are also useful for the monitoring of the immune responses in the context of preclinical and clinical trials, e.g. to determine immunogenicity and efficacy of new candidate vaccines.

The invention thus applies to the use of VLPs pseudotyped with a protein from an enveloped virus, as described herein, for the in vitro detection of neutralizing antibodies directed against the enveloped virus in a biological sample from a subject susceptible to be infected with said enveloped virus. Said biological sample may be a biological fluid, such as blood or serum, or a tissue. In a specific embodiment, the enveloped virus is an orthomyxovirus and said antibodies are directed against HA and/or NA influenza proteins.

This method achieves detection of specific antibodies that are neutralizing for cell infection, i.e. those patient antibodies that are effective against virus *in vivo.*

The invention further provides a composition comprising said VLPs in association with a vehicle. The vehicle may be any carrier that, advantageously, does not affect stability of the VLPs.

The below examples illustrate the invention without limiting its scope.

### EXAMPLES

### I. Materials and Methods

### 1. Plasmid DNA and recombinant viral vectors

The following DNA expression plasmids were used:
a. CMV-driven expression vector of MLV retroviral core Gag/Pol,
b. Expression plasmids coding the hemagglutinin (HA) and neuraminidase (NA) from Influenza A virus A/Vietnam/1194/2004 (H5N1, HA GenBank accession n° AY651333, NA n° AY651445), A/HongKong/156/1997 (H5N1, HA n° AF036356, NA n° AF036357), A/Brisbane/59/2007 (H1N1, HA n° ACA28846, NA n° ACA28847), A/New Mexico/04/2009 (H1N1, HA n° ACR08530, NA n° ACR08568), A/England/321/1977 (H3N2, HA n°CAA29337) and A/Uruguay/716/2007-X-175 (H3N2, NA n° ACD47185) under the control of a CMV promoter.
c. Packaging competent (Psi+) MLV-based plasmids containing either a CMV-GFP or a CMV-Firefly luciferase internal transcriptional unit.

### 2. Construction of MLV retroviral double-reporter vector

The double-reporter vector consists in a packaging competent (Psi+) MLV-based vector that encodes CMV-driven expression of a single polyprotein GFP-2A-Luciferase further cleaved at the level of a peptide sequence introduced between the two proteins, the small 2A polypeptide cleavage site from FMDV **(see Figures 1A and 1B)**. Briefly, the double-reporter retroviral vector was generated by inserting a synthesized DNA fragment containing a 5' BamHI cloning site, the coding sequence for enhanced-GFP (eGFP, accession n° P42212) cloned in frame with 72 nucleotides coding FMDV 2A cleavage site (accession n° P03305) and with codon-optimized sequence of *firefly* luciferase (accession n° P08659), and a 3' Xhol cloning site (**SEQ ID NO: 1**) into the BamHI and Xhol digested commercial MLV-based retroviral vector (pQCXIX,Clontech). The vector allows expression of GFP-2A-Luciferase polyprotein (**SEQ ID NO: 2**) under the control of a CMV promoter in target cells transduced with MLV-based pseudoparticles.

### 3. Production of infectious particles pseudotyped with Influenza A virus envelopes

Pseudoparticles were generated essentially as described previously (Bartosch, et al 2003, J. Exp. Med. 197:633-642; Hsu, et al, 2003, Proc. Natl. Acad. Sci.USA 100:7271-7276.). Briefly, HEK-293T human embryo kidney cells (ATCC-CRL-11268) were co-transfected with expression vectors encoding the viral components using Calphos Mammalian Transfection Kit (Clontech), i.e.,
i) a CMV-driven expression vector of MLV retroviral core Gag/Pol;
ii) either two MLV retroviral transfer vectors carrying a marker gene encoding the luciferase *firefly* (F-Luc) and the GFP, respectively, or the double-reporter vector carrying the GFP-2A-Luciferase cassette;
iii) plasmid(s) encoding viral envelopes of interest.

Non-pseudotyped particles were prepared by co-transfection of HEK-293T cells with the expression vectors encoding Gag-Pol and the luciferase *firefly* and/or *GFP.*

HEK-293T cell supernatants containing infectious pseudo-particles were harvested 48 h or 72 h after transfection, filtered through 0.45 µm-pore-size membranes, and frozen at -80°C until further analysis.

### 4. Transduction assay and determination of retroviral particle infectious titer

Evaluation of the specific pseudoparticle infectivity was assessed in a one-round infection process, using target cell lines permissive for infection with particles expressing envelope of virus of interest (**Figure 2A** **and** **2B**).

To determine the transduction efficiency and infection titer of pseudotyped retroviral particles, target cells were seeded at a density of 6500 cells per well in 96-well microplates (BD-bioscience) one day before transduction. Target cells used were MDCK (ATCC-CCL-34) or TE-671 (ATCC-CRL-8805) to monitor infection by Influenza pseudoparticles (FLUpp). They were grown as recommended by the American Type Culture Collection in complete medium composed of DMEM supplemented with 10% fetal calf serum (FCS), 2mM L-Glutamin, 100 U/mL Penicillin/Sreptomycin (all from Invitrogen).

Serial dilutions of particle preparation (20 µL) were added to the target cells, and the cultures were incubated for four hours at 37°C, 5%CO2. The vector-containing medium was then replaced with normal culture medium and the cells were incubated for 48 to 72 hours at 37°C. The percentage of GFP-positive cells was determined by flow cytometry on a FACS'calibur (Becton-Dickinson) equipped with the High Throughput Sampler (HTS) option for microplate analysis, following treatment of the transduced cells in 0.05 % trypsin. Infection titer, provided as transducing units (TU)/mL, was calculated by using the formula: Titer = % of infection x (cell number/100) x 50 x d, where % of infection is the percentage of GFP-positive cells as determined by flow cytometry analysis using dilutions of the viral supernatant that transduced preferably between 5% and 10% of GFP-positive cells and d is the corresponding dilution factor of the viral supernatant (Negre et al., Gene Therapy, 2000, 7(19):1613-23).

### 5. Neutralization assay

Pseudoparticles used at standardized dilutions according to their infectious titer were preincubated for 45min at 37°C with dilutions of heat-inactivated (30min at 56°C) sera or antibodies to be tested. The pre-incubation mixture was then added to the target cells (TE671 or MDCK) seeded the day before in 96-well white microplates (NUNC). The plates were incubated for 4 hours at 37°C, 5%CO2. The supernatants were removed and replaced by fresh complete medium as previously (DMEM supplemented with 10% FCS, Glutamine 2mM and Peni/Strepto 100 U/mL GIBCO). 48 to 72 hours post-infection, Luciferase activity in infected cells was measured by using the Bright-Glo Luciferase assay system (Promega) according to manufacturer recommendations and a CentroXS 96-well microplate luminometer (Berthold). Data were expressed in RLU for Relative Luminescence Unit of *firefly* Luciferase. Samples were tested in triplicates and results were expressed as mean +/- SD of RLU or % of neutralisation.

### II. Results

### EXAMPLE 1: Infectivity of Influenza A pseudoparticles expressing two reporter genes GFP and Luciferase in target cells: use of GFP expression in target cells to determine the infectious titer.

Measurement of transduction efficiency of target cells by pseudotyped retroviral particles is essential for standardization of neutralization assays. To do that, avian influenza pseudoparticles (avian FLUpp) were produced in HEK-293T cells by transfection with two MLV-based transfer vectors carrying the GFP and firefly luciferase reporter gene, respectively, and by expression of HA and NA proteins from Influenza virus A/Vietnam/1194/2004 (H5N1) onto retroviral core proteins derived from MLV. After 48 hours transfection, serial dilutions of HEK-293T cell supernatant were titrated on TE671 target cells. As shown in **Figure 3****,** infection with avian FLUpp of target cells was efficient as determined by measuring the % of GFP positive cells by flow cytometry (A) and by measuring the luciferase activity in target cells by luminescence (B). As control, no specific infection was detected by measuring the GFP or luciferase activity in TE671 cells incubated with dilutions of 293T cell supernatants containing pseudoparticles produced without envelope HA and NA (no Env) or with HA alone. Results obtained with measurement of the GFP reporter allowed calculating an infectious titer of 9.0 x 10⁶ TU/mL for this H5N1-FLUpp preparation. These results indicated that the efficiency of infection of target cells can be determined by measuring activity of the luciferase and that infectious titer can be determined by analysis of GFP-positive target cells for a same batch of pseudoparticles.

### EXAMPLE 2: The neutralizing activity of a sample is dependent on the infectious titer of the pseudotyped pseudoparticles.

To investigate further whether determination of infectious titer of pseudoparticles used in an experiment is required for standardization of neutralization assay, titration of an antiInfluenza H5 neutralizing antibody (mAb 7C2, US Biological) was assessed against different dilutions (corresponding to different infectious titers) of the H5N1-FLUpp preparation previously titrated in Example 1. Measurement of the luciferase activity in target cells (**Figure 4**) showed that the dose response curve of neutralization by mAb 7C2 was shifted by using increasing titers of H5N1-FLUpp. Determination of the concentration of mAb 7C2 inhibiting 50% of infection of target cells by H5N1-FLUpp (IC50) demonstrated the IC50 could vary from 8 to 80 ng/ml when using 0.06 x 10⁶ to 1.65 x 10⁶ TU/ml of H5N1-FLUpp. Therefore, neutralization activity of mAb 7C2 was depending on infectious titer of the avian FLUpp used in the assay. This result indicated that precise determination of infectious titer and use of a defined amount of pseudotyped retroviral particles are required for standardization of neutralization assays, in particular for comparison of the neutralization activity of sample(s) against different preparations of pseudoparticles.

### EXAMPLE 3: Use of standardized Influenza A pseudoparticles expressing GFP and Luciferase to monitor the induction of Influenza neutralizing antibody responses in mice immunized with different vaccines.

Immunogenicity study in mice of DNA candidate vaccines can be tested with standardized neutralization assays (**Figures 5A****,** **5B**). In this study, three DNA candidate vaccines against Influenza HSN1 A/Vietnam/1194/2004 virus have been tested in mice. Therefore, the study contained four groups of nine mice:
- A: DNA Candidate vaccine 1
- B: DNA Candidate vaccine 2
- C: DNA Candidate vaccine 3
- D: Naive

Three injections were done at two days interval (DO, D2, and D4). Each DNA preparation (0,5 µg each) was coated onto gold beads before intradermal injection using Gene Gun technology. Samples of blood were taken just before immunizations at day 0 (DO), and at Day 10 (D10) and Day 30 (D30). Neutralization assays using H5N1-FLUpp (A/Vietnam/1194/2004) were performed to assess the reactivity of each serum against H5N1 virus (**Figure 5A**).

First of all, infectivity of H5N1-FLUpp was assessed on TE671 cells, as previously described. Thus, infectious titer of up to 2.7 x 10⁷ TU/mL was obtained for the H5N1-FLUpp. This result showed that H5N1-FLUpp is highly infectious. For standardization, neutralization assays were performed using H5N1-FLUpp at 10⁵ TU/mL, corresponding to 15% of target cells infection.

After that, the efficacy of infection in the presence or absence of mice serum was determined by measuring luciferase activity in target cells. As shown in **Figure 5A**, no or only weak neutralization of H5N1-FLUpp could be detected for all sera at D0. However, most of the sera from immunized mice (Group A, B and C) could neutralize the infectivity of H5N1-FLUpp at D10 and D30, in contrast to sera derived from naïve mice (Group D). Variable levels of neutralization were detected depending on the candidate vaccine injected in mice and an increase of neutralization level was observed between D10 and D30.

With those results, neutralization titer could be determined for each serum and each DNA candidate vaccine could be compared for their potency (**Figure 5B**).

Thus, neutralization assays using infectious pseudoparticles at a defined infectious titer allows the follow-up study of immunogenicity in mice and evaluation of candidate vaccines to induce neutralizing antibodies.

### EXAMPLE 4: Use of a packaging competent MLV-based double-reporter vector for optimal standardization and evaluation of the capacity of human serum to neutralize infection by several strains of influenza viral particles.

Infectious retroviral particles incorporating the double reporter vector encoding the eGFP-2A-Luc polyprotein were pseudotyped with the HA and NA from influenza A/New Mexico/2009 (pandemic H1N1), or A/Brisbane/59/2007 (seasonal H1N1), or A/England/321/1977 (seasonal H3N2), or A/HongKong/156/1997 (avian flu, H5N1) virus. FLUpp were further titrated by serial dilution on MDCK cells and the % of GFP positive cells was measured by flow cytometry (**Figure 6**) in order to calculate the infection titer of each FLUpp preparation. Infectious titers varied from 2.7 x10⁴ to 8.6 x 10⁵ TU/mL. Neutralization assays were then performed by using the same infectious titer of 1 x 10⁴ TU/mL for each FLUpp. The Luciferase activity was measured in target cells and the % of neutralization relative to incubation in the absence of antiserum was calculated for each sample. In such conditions, reference control antisera obtained by immunization of animals with a determined HA showed specific neutralization activities (**Figure 7**). In contrast, a pool of human sera recommended as standard for antibody to influenza H1N1v (pandemic variant 2009) virus (NIBSC 09/194) showed polyspecific neutralization activity against the FLUpp pseudotyped with the human pandemic (H1N1-2009) and seasonal (H1N1-2007 and H3N2-1977) influenza, but had no activity against the FLUpp pseudotyped with the avian flu H5N1 (**Figure 8**).

## Claims

1. An *in vitro* method for evaluating a test substance for its ability to modulate, preferably to inhibit, infectivity of an enveloped virus, which method comprises
a) contacting a host cell with a population of recombinant pseudotyped retrovirus particles comprising (i) a replication-defective genome of a retrovirus, (ii) core protein(s) of said retrovirus, and (iii) an envelope that comprises at least one protein from an enveloped virus, wherein said population altogether carries at least two R1 and R2 reporter genes, wherein R1 and R2 are distinct, and measuring infectious titer of said particles by means of the R1 reporter gene;
b) adjusting the dilution of the population of retrovirus particles showing the infectious titer measured in step (a) to reach a defined infectious titer suitable for measuring efficacy of infection, and, in the presence or the absence of the test substance, contacting a host cell with said population of retrovirus particles at said defined infectious titer and measuring efficacy of infection of said cell by said retrovirus particles by means of the R2 reporter gene; and
c) comparing the efficacy of infection in the presence of the test substance with efficacy of infection in the absence of the test substance, whereby the ability of the test substance to modulate infectivity of the retrovirus particle is determined and reflects the ability of the test substance to modulate infectivity of the enveloped virus.

2. The method of claim 1, wherein the reporter genes R1 and R2 are carried by a sole retrovirus genome and preferably are operably linked.

3. The method of claim 1, wherein the reporter genes R1 and R2 are carried by two distinct retrovirus genomes.

4. The method of any of claims 1 to 3, wherein the retrovirus particles comprise an envelope that comprises at least one protein from an enveloped virus that is heterologous to said retrovirus.

5. The method of any of claims 1 to 4, wherein said reporter gene R1 encodes a gene product suitable for measuring infectious titer of said retrovirus particles, by counting the number of cells transduced by the particles, such as an enzyme, a chromogenic agent, or a fluorescent agent.

6. The method of any of claims 1 to 5, wherein said reporter gene R2 encodes a gene product suitable for measuring the efficacy of infection of said cell by said retrovirus vector, such as an enzyme, a chromogenic agent, a luminescent agent, or a fluorescent agent.

7. The method of any of claims 1 to 6, wherein said reporter gene R1 encodes Green Fluorescent Protein (GFP) and said reporter gene R2 encodes Luciferase.

8. A recombinant pseudotyped retrovirus particle comprising (i) a replication-defective genome of a retrovirus, (ii) core protein(s) of said retrovirus, and (iii) an envelope that comprises at least one protein from an enveloped virus wherein said genome includes at least one reporter gene R1 and at least one reporter gene R2, wherein R1 and R2 are distinct and preferably are operably linked.

9. The particle of claim 9, wherein said reporter genes R1 and R2 are in the form of a monocistronic RNA or of a bicistronic RNA, preferably comprising an internal ribosome entry site (IRES) between the two reporter genes.

10. The particle of any of claims 9 or 10, wherein said reporter genes R1 and R2 encode a single polyprotein that comprises a site of cleavage, such as a small 2A polypeptide cleavage site of the foot-and-mouth disease virus (FMDV), located between the translation product of R1 and the translation product of R2.

11. The particle of any of claims 9 to 11, wherein said reporter genes R1 and R2 encode a single fusion protein.

12. The particle of any of claims 9 to 11, wherein the genome is a genome from a retrovirus selected from the group consisting of MLV, ALV, RSV, MPMV, HIV-,1 HIV-2, SIV, EIAV, CAEV, and HFV.

13. The particle of any of claims 9 to 12, wherein said enveloped virus is selected from the group consisting of virus of the family of *Arenaviridae* (e.g. Lassa virus), *Coronaviridae* (e.g. Sever Acute Respiratory Syndrome virus), *Flaviviridae* (e.g. Hepatitis C Virus, Dengue virus, West Nil Virus, Yellow Fever Virus, Tick-Borne Encephalitis virus), *Filoviridae* (e.g. Ebola, Marburg), *Herpesviridae* (e.g. Herpes Simplex Virus, Cytomegalovirus, Epstein-Barr Virus, Varicella Zoster Virus), *Orthomyxoviridae* (e.g. Influenza A and B), *Paramyxoviridae* (e.g. Respiratory Syncytial Virus, Paralnfluenza Virus, PMV, Measles), *Poxviridae* (e.g. Vaccinia, Variola), *Rhabdoviridae* (e.g.Vesicular Sstomatitis Virus, Viral Hemorrhagic Septicemia Virus, Rabies), *Retroviridae* (e.g. HIV and other retroviruses), *Togaviridae* (e.g. Chikungunya, Sindbis, Semliki Forest Virus, Ross River Virus, Eastern Equine Encephalitis Virus) .

14. A composition comprising a population of recombinant pseudotyped retrovirus particles comprising
- particles comprising (i) a replication-defective genome of a retrovirus, (ii) core protein(s) of said retrovirus, and (iii) an envelope that comprises at least one protein from an enveloped virus, wherein said genome includes at least one reporter gene R1,
- and particles comprising (i) a replication-defective genome of said retrovirus, (ii) core protein(s) of said retrovirus, and (iii) an envelope that comprises at least said protein from said enveloped virus, wherein said genome includes at least one reporter gene R2, distinct from R1,
in association with a vehicle.

15. The composition of claim 14, wherein the population further comprises such recombinant pseudotyped retrovirus particles comprising a genome that includes at least one reporter gene R1 and at least one reporter gene R2, distinct from R1, where R1 and R2 are operably linked.


SEQUENCE LISTING

<110>  EPIXIS

<120>  A method for measuring viral infectivity

<130>  B1005

<160>  2

<170>  PatentIn version 3.3

<210>  1
<211>  2469
<212>  DNA
<213>  artificial sequence

<220>
<223>  SEQ ID NO: 1


<220>
<221>  misc_feature
<222>  (1)..(15)
<223>  label="5' \BamHI \cloning \site"
sequence="ggatccgatatcacc"

<220>
<221>  CDS
<222>  (16)..(2454)
<223>  gene="GFP2ALUC"
       note="confers green fluorescence and luciferase activity"

<220>
<221>  misc_feature
<222>  (16)..(732)
<223>  gene="eGFP"
note="eGFP from Aequorea vistoria"

<220>
<221>  misc_feature
<222>  (733)..(804)
<223>  gene="2A"
note="FMDV 2A self-cleaving linker region"

<220>
<221>  misc_feature
<222>  (805)..(2454)
<223>  gene="luciferase"
       note="codon-optimized firefly luciferase from Photinus pyralis"

<220>
<221>  misc_feature
<222>  (2455)..(2469)
<223>  label="3' \XhoI \cloning \site"
sequence="gcggccgcctcgagt"

<400>  1

ggatccgata tcacc atg gtg agc aag ggc gag gag ctg ttc acc ggg gtg 51
Met Val Ser Lys Gly Glu Glu Leu Phe Thr Gly Val
1               5                   10

gtg ccc atc ctg gtc gag ctg gac ggc gac gta aac ggc cac aag ttc 99
Val Pro Ile Leu Val Glu Leu Asp Gly Asp Val Asn Gly His Lys Phe
        15                  20                  25

agc gtg tcc ggc gag ggc gag ggc gat gcc acc tac ggc aag ctg acc 147
Ser Val Ser Gly Glu Gly Glu Gly Asp Ala Thr Tyr Gly Lys Leu Thr
    30                  35                  40

ctg aag ttc atc tgc acc acc ggc aag ctg ccc gtg ccc tgg ccc acc 195
Leu Lys Phe Ile Cys Thr Thr Gly Lys Leu Pro Val Pro Trp Pro Thr
45                  50                  55                  60

ctc gtg acc acc ctg acc tac ggc gtg cag tgc ttc agc cgc tac ccc 243
Leu Val Thr Thr Leu Thr Tyr Gly Val Gln Cys Phe Ser Arg Tyr Pro
                65                  70                  75

gac cac atg aag cag cac gac ttc ttc aag tcc gcc atg ccc gaa ggc 291
Asp His Met Lys Gln His Asp Phe Phe Lys Ser Ala Met Pro Glu Gly
            80                  85                  90

tac gtc cag gag cgc acc atc ttc ttc aag gac gac ggc aac tac aag 339
Tyr Val Gln Glu Arg Thr Ile Phe Phe Lys Asp Asp Gly Asn Tyr Lys
        95                  100                 105

acc cgc gcc gag gtg aag ttc gag ggc gac acc ctg gtg aac cgc atc 387
Thr Arg Ala Glu Val Lys Phe Glu Gly Asp Thr Leu Val Asn Arg Ile
    110                 115                 120

gag ctg aag ggc atc gac ttc aag gag gac ggc aac atc ctg ggg cac 435
Glu Leu Lys Gly Ile Asp Phe Lys Glu Asp Gly Asn Ile Leu Gly His
125                 130                 135                 140

aag ctg gag tac aac tac aac agc cac aac gtc tat atc atg gcc gac 483
Lys Leu Glu Tyr Asn Tyr Asn Ser His Asn Val Tyr Ile Met Ala Asp
                145                 150                 155

aag cag aag aac ggc atc aag gtg aac ttc aag atc cgc cac aac atc 531
Lys Gln Lys Asn Gly Ile Lys Val Asn Phe Lys Ile Arg His Asn Ile
            160                 165                 170

gag gac ggc agc gtg cag ctc gcc gac cac tac cag cag aac acc ccc 579
Glu Asp Gly Ser Val Gln Leu Ala Asp His Tyr Gln Gln Asn Thr Pro
        175                 180                 185

atc ggc gac ggc ccc gtg ctg ctg ccc gac aac cac tac ctg agc acc 627
Ile Gly Asp Gly Pro Val Leu Leu Pro Asp Asn His Tyr Leu Ser Thr
    190                 195                 200

cag tcc gcc ctg agc aaa gac ccc aac gag aag cgc gat cac atg gtc 675
Gln Ser Ala Leu Ser Lys Asp Pro Asn Glu Lys Arg Asp His Met Val
205                 210                 215                 220

ctg ctg gag ttc gtg acc gcc gcc ggg atc act ctc ggc atg gac gag 723
Leu Leu Glu Phe Val Thr Ala Ala Gly Ile Thr Leu Gly Met Asp Glu

225                 230                 235

ctg tac aag gca ccg gtg aaa cag act ttg aat ttt gac ctt ctc aag       771
Leu Tyr Lys Ala Pro Val Lys Gln Thr Leu Asn Phe Asp Leu Leu Lys
        240                 245                 250

ttg gcg gga gac gtg gag tcc aac cct ggg ccc gaa gat gcc aaa aac       819
Leu Ala Gly Asp Val Glu Ser Asn Pro Gly Pro Glu Asp Ala Lys Asn
    255                 260                 265

att aag aag ggc cca gcg cca ttc tac cca ctc gaa gac ggg acc gcc       867
Ile Lys Lys Gly Pro Ala Pro Phe Tyr Pro Leu Glu Asp Gly Thr Ala
    270                 275                 280

ggc gag cag ctg cac aaa gcc atg aag cgc tac gcc ctg gtg ccc ggc       915
Gly Glu Gln Leu His Lys Ala Met Lys Arg Tyr Ala Leu Val Pro Gly
285                 290                 295                 300

acc atc gcc ttt acc gac gca cat atc gag gtg gac att acc tac gcc       963
Thr Ile Ala Phe Thr Asp Ala His Ile Glu Val Asp Ile Thr Tyr Ala
                305                 310                 315

gag tac ttc gag atg agc gtt cgg ctg gca gaa gct atg aag cgc tat      1011
Glu Tyr Phe Glu Met Ser Val Arg Leu Ala Glu Ala Met Lys Arg Tyr
            320                 325                 330

ggg ctg aat aca aac cat cgg atc gtg gtg tgc agc gag aat agc ttg      1059
Gly Leu Asn Thr Asn His Arg Ile Val Val Cys Ser Glu Asn Ser Leu
        335                 340                 345

cag ttc ttc atg ccc gtg ttg ggt gcc ctg ttc atc ggt gtg gct gtg      1107
Gln Phe Phe Met Pro Val Leu Gly Ala Leu Phe Ile Gly Val Ala Val
    350                 355                 360

gcc cca gct aac gac atc tac aac gag cgc gag ctg ctg aac agc atg      1155
Ala Pro Ala Asn Asp Ile Tyr Asn Glu Arg Glu Leu Leu Asn Ser Met
365                 370                 375                 380

ggc atc agc cag ccc acc gtc gta ttc gtg agc aag aaa ggg ctg caa      1203
Gly Ile Ser Gln Pro Thr Val Val Phe Val Ser Lys Lys Gly Leu Gln
                385                 390                 395

aag atc ctc aac gtg caa aag aag cta ccg atc ata caa aag atc atc      1251
Lys Ile Leu Asn Val Gln Lys Lys Leu Pro Ile Ile Gln Lys Ile Ile
            400                 405                 410

atc atg gat agc aag acc gac tac cag ggc ttc caa agc atg tac acc      1299
Ile Met Asp Ser Lys Thr Asp Tyr Gln Gly Phe Gln Ser Met Tyr Thr
        415                 420                 425

ttc gtg act tcc cat ttg cca ccc ggc ttc aac gag tac gac ttc gtg      1347
Phe Val Thr Ser His Leu Pro Pro Gly Phe Asn Glu Tyr Asp Phe Val
    430                 435                 440

ccc gag agc ttc gac cgg gac aaa acc atc gcc ctg atc atg aac agt      1395
Pro Glu Ser Phe Asp Arg Asp Lys Thr Ile Ala Leu Ile Met Asn Ser
445                 450                 455                 460

agt ggc agt acc gga ttg ccc aag ggc gta gcc cta ccg cac cgc acc     1443
Ser Gly Ser Thr Gly Leu Pro Lys Gly Val Ala Leu Pro His Arg Thr
                465                 470                 475

gct tgt gtc cga ttc agt cat gcc cgc gac ccc atc ttc ggc aac cag     1491
Ala Cys Val Arg Phe Ser His Ala Arg Asp Pro Ile Phe Gly Asn Gln
            480                 485                 490

atc atc ccc gac acc gct atc ctc agc gtg gtg cca ttt cac cac ggc     1539
Ile Ile Pro Asp Thr Ala Ile Leu Ser Val Val Pro Phe His His Gly
        495                 500                 505

ttc ggc atg ttc acc acg ctg ggc tac ttg atc tgc ggc ttt cgg gtc     1587
Phe Gly Met Phe Thr Thr Leu Gly Tyr Leu Ile Cys Gly Phe Arg Val
    510                 515                 520

gtg ctc atg tac cgc ttc gag gag gag cta ttc ttg cgc agc ttg caa     1635
Val Leu Met Tyr Arg Phe Glu Glu Glu Leu Phe Leu Arg Ser Leu Gln
525                 530                 535                 540

gac tat aag att caa tct gcc ctg ctg gtg ccc aca cta ttt agc ttc     1683
Asp Tyr Lys Ile Gln Ser Ala Leu Leu Val Pro Thr Leu Phe Ser Phe
                545                 550                 555

ttc gct aag agc act ctc atc gac aag tac gac cta agc aac ttg cac     1731
Phe Ala Lys Ser Thr Leu Ile Asp Lys Tyr Asp Leu Ser Asn Leu His
            560                 565                 570

gag atc gcc agc ggc ggg gcg ccg ctc agc aag gag gta ggt gag gcc     1779
Glu Ile Ala Ser Gly Gly Ala Pro Leu Ser Lys Glu Val Gly Glu Ala
        575                 580                 585

gtg gcc aaa cgc ttc cac cta cca ggc atc cgc cag ggc tac ggc ctg     1827
Val Ala Lys Arg Phe His Leu Pro Gly Ile Arg Gln Gly Tyr Gly Leu
    590                 595                 600

aca gaa aca acc agc gcc att ctg atc acc ccc gaa ggg gac gac aag     1875
Thr Glu Thr Thr Ser Ala Ile Leu Ile Thr Pro Glu Gly Asp Asp Lys
605                 610                 615                 620

cct ggc gca gta ggc aag gtg gtg ccc ttc ttc gag gct aag gtg gtg     1923
Pro Gly Ala Val Gly Lys Val Val Pro Phe Phe Glu Ala Lys Val Val
                625                 630                 635

gac ttg gac acc ggt aag aca ctg ggt gtg aac cag cgc ggc gag ctg     1971
Asp Leu Asp Thr Gly Lys Thr Leu Gly Val Asn Gln Arg Gly Glu Leu
            640                 645                 650

tgc gtc cgt ggc ccc atg atc atg agc ggc tac gtt aac aac ccc gag     2019
Cys Val Arg Gly Pro Met Ile Met Ser Gly Tyr Val Asn Asn Pro Glu
        655                 660                 665

gct aca aac gct ctc atc gac aag gac ggc tgg ctg cac agc ggc gac     2067
Ala Thr Asn Ala Leu Ile Asp Lys Asp Gly Trp Leu His Ser Gly Asp
    670                 675                 680

atc gcc tac tgg gac gag gac gag cac ttc ttc atc gtg gac cgg ctg     2115
Ile Ala Tyr Trp Asp Glu Asp Glu His Phe Phe Ile Val Asp Arg Leu

685                 690                 695                 700

aag agc ctg atc aaa tac aag ggc tac cag gta gcc cca gcc gaa ctg      2163
Lys Ser Leu Ile Lys Tyr Lys Gly Tyr Gln Val Ala Pro Ala Glu Leu
                705                 710                 715

gag agc atc ctg ctg caa cac ccc aac atc ttc gac gcc ggg gtc gcc      2211
Glu Ser Ile Leu Leu Gln His Pro Asn Ile Phe Asp Ala Gly Val Ala
            720                 725                 730

ggc ctg ccc gac gac gat gcc ggc gag ctg ccc gcc gca gtc gtc gtg      2259
Gly Leu Pro Asp Asp Asp Ala Gly Glu Leu Pro Ala Ala Val Val Val
        735                 740                 745

ctg gaa cac ggt aaa acc atg acc gag aag gag atc gtg gac tat gtg      2307
Leu Glu His Gly Lys Thr Met Thr Glu Lys Glu Ile Val Asp Tyr Val
    750                 755                 760

gcc agc cag gtt aca acc gcc aag aag ctg cgc ggt ggt gtt gtg ttc      2355
Ala Ser Gln Val Thr Thr Ala Lys Lys Leu Arg Gly Gly Val Val Phe
765                 770                 775                 780

gtg gac gag gtg cct aaa gga ctg acc ggc aag ttg gac gcc cgc aag      2403
Val Asp Glu Val Pro Lys Gly Leu Thr Gly Lys Leu Asp Ala Arg Lys
                785                 790                 795

atc cgc gag att ctc att aag gcc aag aag ggc ggc aag atc gcc gtg      2451
Ile Arg Glu Ile Leu Ile Lys Ala Lys Lys Gly Gly Lys Ile Ala Val
            800                 805                 810

tga gcggccgcct cgagt                                                 2469


<210>  2
<211>  812
<212>  PRT
<213>  artificial sequence

<220>
<223>  Synthetic Construct

<400>  2

Met Val Ser Lys Gly Glu Glu Leu Phe Thr Gly Val Val Pro Ile Leu
1               5                   10                  15


Val Glu Leu Asp Gly Asp Val Asn Gly His Lys Phe Ser Val Ser Gly
            20                  25                  30


Glu Gly Glu Gly Asp Ala Thr Tyr Gly Lys Leu Thr Leu Lys Phe Ile
        35                  40                  45


Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys Ala
225                 230                 235                 240

Pro Val Lys Gln Thr Leu Asn Phe Asp Leu Leu Lys Leu Ala Gly Asp
                245                 250                 255

Val Glu Ser Asn Pro Gly Pro Glu Asp Ala Lys Asn Ile Lys Lys Gly
            260                 265                 270

Pro Ala Pro Phe Tyr Pro Leu Glu Asp Gly Thr Ala Gly Glu Gln Leu
        275                 280                 285

His Lys Ala Met Lys Arg Tyr Ala Leu Val Pro Gly Thr Ile Ala Phe

290                 295                 300

Thr Asp Ala His Ile Glu Val Asp Ile Thr Tyr Ala Glu Tyr Phe Glu
305                 310                 315                 320

Met Ser Val Arg Leu Ala Glu Ala Met Lys Arg Tyr Gly Leu Asn Thr
                325                 330                 335

Asn His Arg Ile Val Val Cys Ser Glu Asn Ser Leu Gln Phe Phe Met
            340                 345                 350

Pro Val Leu Gly Ala Leu Phe Ile Gly Val Ala Val Ala Pro Ala Asn
        355                 360                 365

Asp Ile Tyr Asn Glu Arg Glu Leu Leu Asn Ser Met Gly Ile Ser Gln
    370                 375                 380

Pro Thr Val Val Phe Val Ser Lys Lys Gly Leu Gln Lys Ile Leu Asn
385                 390                 395                 400

Val Gln Lys Lys Leu Pro Ile Ile Gln Lys Ile Ile Ile Met Asp Ser
                405                 410                 415

Lys Thr Asp Tyr Gln Gly Phe Gln Ser Met Tyr Thr Phe Val Thr Ser
            420                 425                 430

His Leu Pro Pro Gly Phe Asn Glu Tyr Asp Phe Val Pro Glu Ser Phe
        435                 440                 445

Asp Arg Asp Lys Thr Ile Ala Leu Ile Met Asn Ser Ser Gly Ser Thr
    450                 455                 460

Gly Leu Pro Lys Gly Val Ala Leu Pro His Arg Thr Ala Cys Val Arg
465                 470                 475                 480

Phe Ser His Ala Arg Asp Pro Ile Phe Gly Asn Gln Ile Ile Pro Asp
                485                 490                 495

Thr Ala Ile Leu Ser Val Val Pro Phe His His Gly Phe Gly Met Phe
            500                 505                 510

Thr Thr Leu Gly Tyr Leu Ile Cys Gly Phe Arg Val Val Leu Met Tyr
        515                 520                 525

Arg Phe Glu Glu Glu Leu Phe Leu Arg Ser Leu Gln Asp Tyr Lys Ile
    530                 535                 540

Gln Ser Ala Leu Leu Val Pro Thr Leu Phe Ser Phe Phe Ala Lys Ser
545                 550                 555                 560

Thr Leu Ile Asp Lys Tyr Asp Leu Ser Asn Leu His Glu Ile Ala Ser
                565                 570                 575

Gly Gly Ala Pro Leu Ser Lys Glu Val Gly Glu Ala Val Ala Lys Arg
            580                 585                 590

Phe His Leu Pro Gly Ile Arg Gln Gly Tyr Gly Leu Thr Glu Thr Thr
        595                 600                 605

Ser Ala Ile Leu Ile Thr Pro Glu Gly Asp Asp Lys Pro Gly Ala Val
    610                 615                 620

Gly Lys Val Val Pro Phe Phe Glu Ala Lys Val Val Asp Leu Asp Thr
625                 630                 635                 640

Gly Lys Thr Leu Gly Val Asn Gln Arg Gly Glu Leu Cys Val Arg Gly
                645                 650                 655

Pro Met Ile Met Ser Gly Tyr Val Asn Asn Pro Glu Ala Thr Asn Ala
            660                 665                 670

Leu Ile Asp Lys Asp Gly Trp Leu His Ser Gly Asp Ile Ala Tyr Trp
        675                 680                 685

Asp Glu Asp Glu His Phe Phe Ile Val Asp Arg Leu Lys Ser Leu Ile
    690                 695                 700

Lys Tyr Lys Gly Tyr Gln Val Ala Pro Ala Glu Leu Glu Ser Ile Leu
705                 710                 715                 720

Leu Gln His Pro Asn Ile Phe Asp Ala Gly Val Ala Gly Leu Pro Asp
                725                 730                 735

Asp Asp Ala Gly Glu Leu Pro Ala Ala Val Val Val Leu Glu His Gly
            740                 745                 750

Lys Thr Met Thr Glu Lys Glu Ile Val Asp Tyr Val Ala Ser Gln Val

755                 760                 765

Thr Thr Ala Lys Lys Leu Arg Gly Gly Val Val Phe Val Asp Glu Val
    770                 775                 780

Pro Lys Gly Leu Thr Gly Lys Leu Asp Ala Arg Lys Ile Arg Glu Ile
785                 790                 795                 800

Leu Ile Lys Ala Lys Lys Gly Gly Lys Ile Ala Val
                805                 810